# EUROPEAN PATENT APPLICATION

(11) **EP 4 223 869 A1**
(43) Date of publication of application: **09.08.2023**
(21) Application number: 22382096.0
(22) Date of filing: 05.02.2022
(51) Int. Cl.: C12N 5/071, A61L 27/38, A61L 27/60, B33Y 80/00

(54) **BIOFABRICATION OF A TRI-LAYERED 3D-BIOPRINTED CSC-BASED MALIGNANT MELANOMA MODEL**

(71) Applicant: Universidad de Granada, 18071 Granada (ES)
(72) Inventor: MARCHAL CORRALES, Juan Antonio, 18016 Granada (ES); JIMÉNEZ GONZÁLEZ, Gema, 18016 Granada (ES); LÓPEZ DE ANDRÉS, Julia, 18016 Granada (ES)
(74) Representative: Hoffmann Eitle

(57) **Abstract**

The present invention relates to the field of biomaterials. Particularly, the present invention relates to a 3D bioprinted melanoma model, methods to produce it hydrogel and medical thereof.

## Description

### TECHNICAL FIELD

The present invention relates to the field of biomaterials. Particularly, the present invention relates to a 3D bioprinted melanoma model, methods to produce it hydrogel and medical thereof.

### BACKGROUND ART

Malignant melanoma (MM), the deadliest form of skin cancer, is currently a major public health concern, due to its rate of increase is higher than for any other solid cancer types, with more than 300,000 new diagnosed cases in 2020 (GLOBOCAN). Despite the most prevalent types of cancer share common characteristics such as high proliferation rates or invasion capability, each one has a unique tumor microenvironment (TME). Apart from tumor cells, TME is composed of multipotent stromal cells/mesenchymal stem cells (MSCs), fibroblasts (FBs), blood vessels, endothelial precursor cells, immune cells, and secreted factors such as cytokines and growth factors 3. Recent findings indicate that these microenvironment features are crucial for the recapitulation of native tumor behaviour.

Traditionally, cancer research has been based on two-dimensional (2D) culture models that do not represent this TME, either mimetic cell-cell or cell-matrix interactions. Another limitation is the lack of good vascularization and many of the known characteristics of tumors in vivo, so they cannot reproduce tumor complexity, restricting their use as biomimetic models in cancer research. Three dimensional (3D) cancer models including a biomimetic extracellular matrix (ECM) have achieved more accurate representations of cancer tissues in terms of TME and biological behaviour, essential for developing early diagnosis and treatment strategies for cancer. Regarding this 3D culture, 3D bioprinting represents the optimal technique to obtain a tumor equivalent, since the heterogeneity and complexity of TME can be replicated by co-printing several cell types, ECM, and biomolecules laden in different bioinks. Moreover, 3D bioprinting allows overcome difficulties associated with current 3D in vitro models, as limited vascularization, lack of well-organized spatial distribution of the biological components or their oversimplified structure. Microextrusion-based 3D bioprinting is the most widely used strategy in current research for tissue biofabrication, since it provides good compatibility with photo, chemical and thermal crosslinkable hydrogels of very different viscosities, with high cell density, and allowing cells viability and proliferation at a reasonable cost. Hydrogels provide perfect soft material systems to mimic native ECM microenvironments and offer more efficient, scalable and repeatable 3D models. Moreover, gel embedding could help for accelerating cancer cell proliferation rate. Collagen type I is the most common hydrogel matrix for skin and tumor 3D modelling, as it supports cell attachment and migration allowing growth and function of several cells' types. When used for 3D bioprinting, collagen can be combined with other ECM components such as hyaluronic acid, in order to obtain more printable bioinks.

To recreate and bioprint a reliable malignant melanoma model not only the bioink but also the structure has to be carefully designed, as the construct has to replicate the multi-layer structure of this tumor type. Skin is a very complex organ, composed of different cell types and organized in three layers (epidermis, dermis and hypodermis). 3D bioprinting has been applied to successfully engineer the stratified human skin by printing their respective cells types in combination with compatible ECM materials, creating biomimetic models that resemble human skin and allowing to bridge the gap between in vitro and clinical testing.

Understanding the underlying biology of tumor initiation and progression is the first step to successful progress in the development of new and efficient cancer therapies, but this is only possible if we are able to mimic the tumour environment in the skin. The present invention provides a 3D malignant melanoma model that reproduces the three layers structure of the skin and that comprises cancer stem cells, resembling the structure and composition of the in vivo model of malignant melanoma.

### BRIEF DESCRIPTION OF DRAWINGS

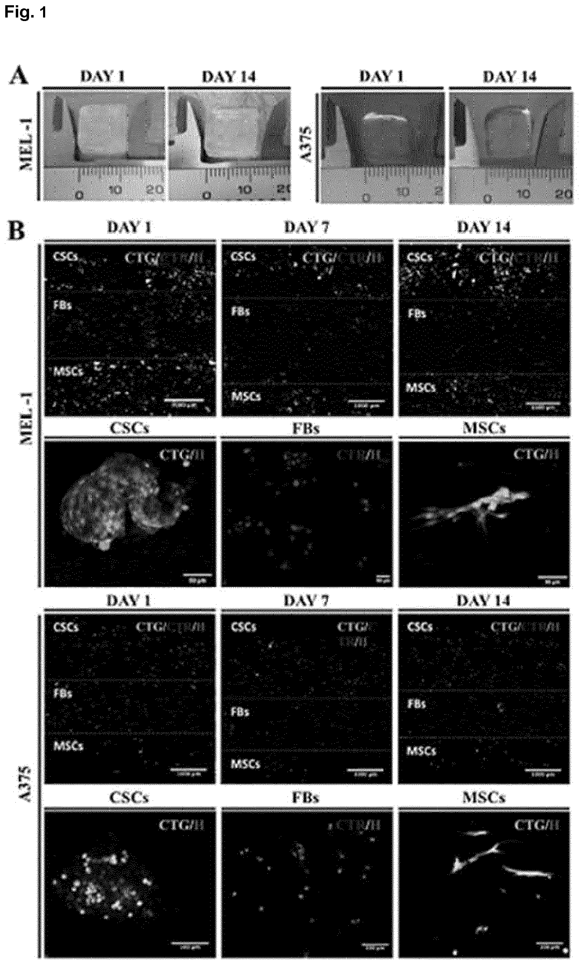

**Fig. 1****: Cellular structure of the three-layered biofabricated MM models.** (A) Images of a trilaminar hydrogel taken at days 1 and 14 after bioprinting. (B) Confocal representative images of 10z stacks obtained by confocal microscopy showing a cross-section of the printed 3D Mel-1 CSCs- and A375 CSCs-based MM models after 1, 7 and 14 days in culture. Scale bar: 1000µm; volume height 80µm. Below the cross-section images, micrographs of the different cell types embedded in the MM biofabricated models taken at day 7 (20x). Scale bars: 50 µm (Mel-1 model) and 100 µm (A375 model).

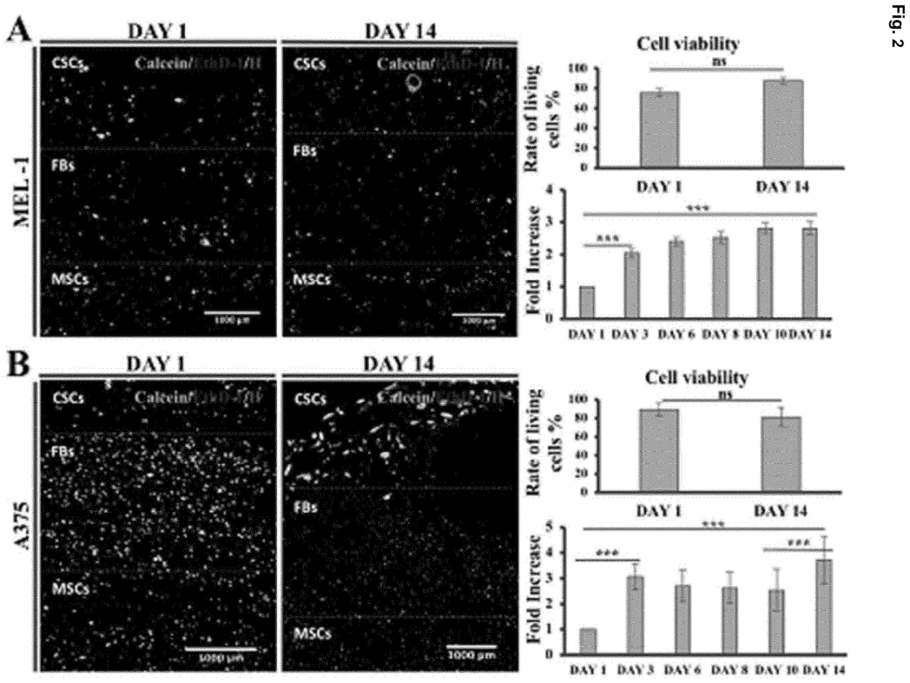

**Fig. 2****: Cell viability and proliferation assays of the three-layered biofabricated MM models.** Confocal representative images of 10z stacks at days 1 and 14 from the CSCs-FBS-MSCs-laden hydrogel of (A) Mel-1 CSCs MM model and (B) A375 CSCs MM model. Scale bar: 1000µm. Graphics show quantification of live/dead cells percent at days 1 and 14 with no significant differences on living cells fraction (n=4; p> 0,05) and fold changes in proliferation of cells (performed with Alamarblue^{®}) embedded in the trilaminar hydrogels at days 1,3,6,8,10 and 14, respectively (n=4; p<0,005).

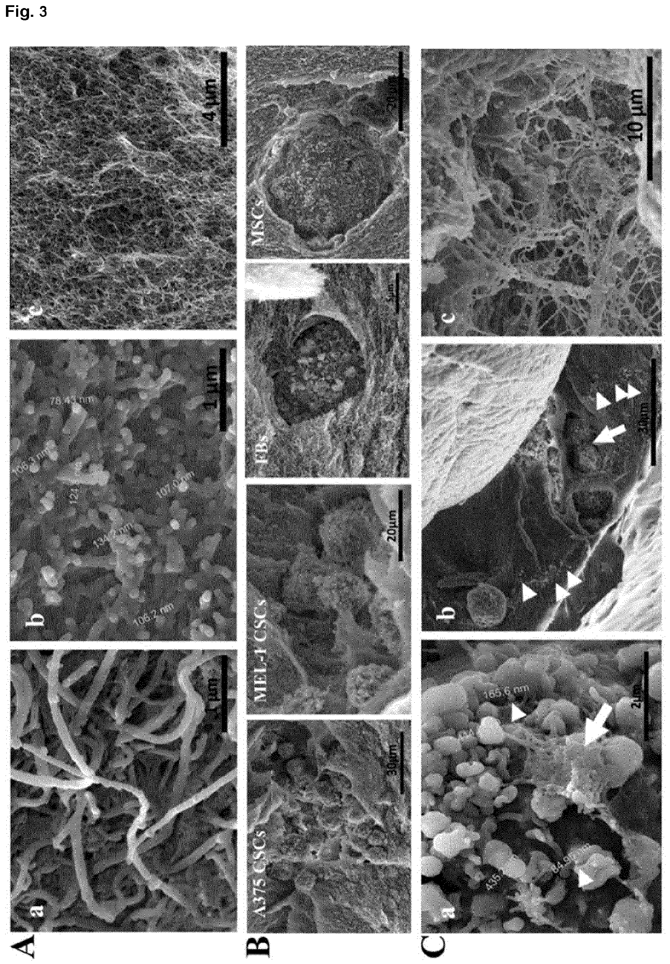

**Fig. 3****: ESEM images of cell-free and cell-laden trilaminar hydrogels.** (A) Cell-free hydrogel fibers on the surface (A1) and hydrogel fiber network observed transversely (A2-3). (B) A375 CSCs, Mel-1 CSCs, MSCs and FBs embedded in the three-layered hydrogel. (C) Cellular activity samples including secretion of different sized microvesicles and exosomes.

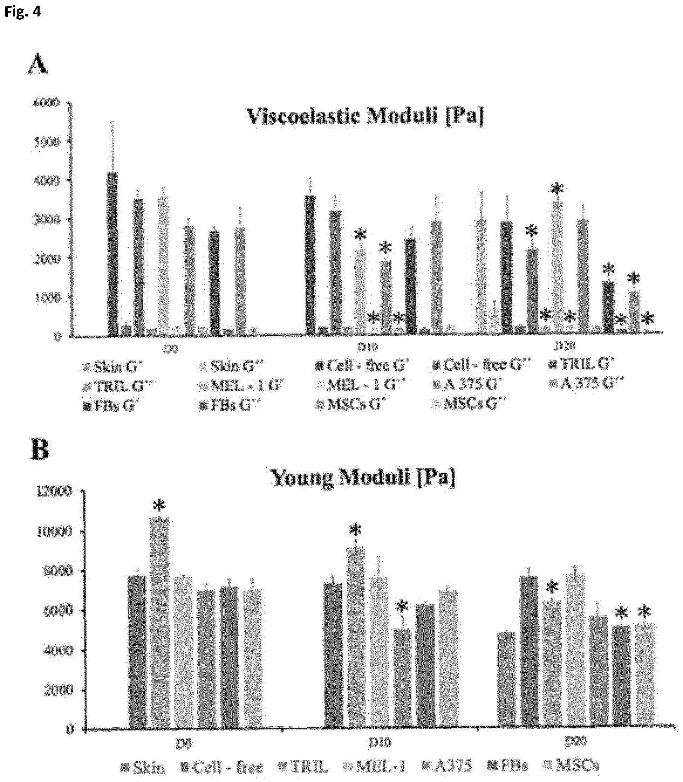

**Fig. 4****: Rheological analysis of cell-free and cell-laden bioprinted hydrogels**. Viscoelastic moduli (A) and Young moduli (B) of agarose-collagen type I hydrogels containing cell-free layers, only one cell-laden layer: MSCs-layer, FBs-layer, CSCs spheres-layer (composed of Mel-1 or A375 cells) or containing the three cell-laden layers together (MSCs, FBs, and CSCs spheres-layers), called trilaminar after 0, 10 and 20 days on culture.

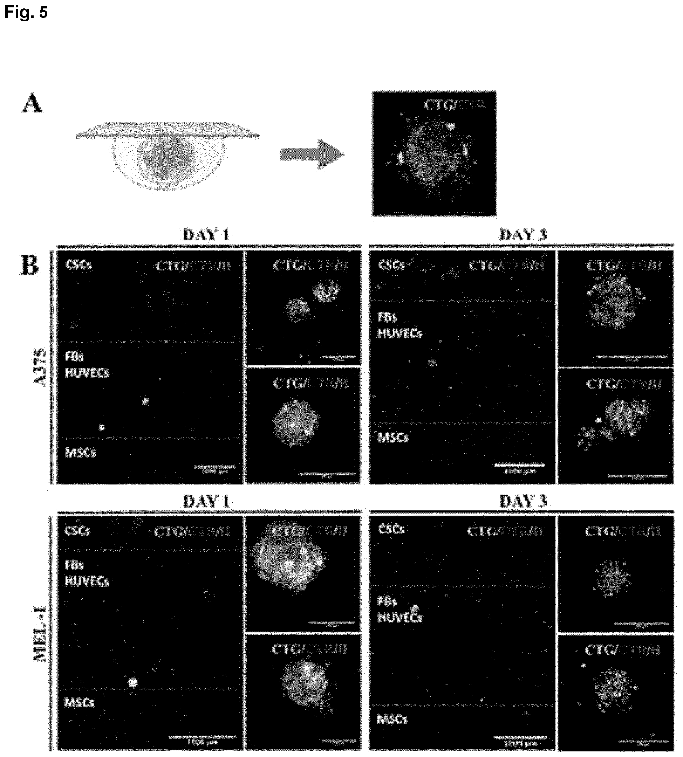

**Fig. 5****: Induction of vascularization in the dermal layer.** (A) Schematic representation and confocal image of a cross section of one HUVECs-FBs spheroid obtained by hanging drop strategy (B) Confocal representative images showing a cross section of the A375 and Mel-1 melanoma trilaminar hydrogels, where spheroids of HUVECs-FBs were bioprinted on the dermal layer. On the right of the cross-section images are shown micrographs of the HUVECs-FBs spheroids embedded in the melanoma models. A375 and Mel-1 CSCs, MSCs and HUVECs were stained with CellTracker Red CMFDA (red) and FBs with CellTracker Green CMFDA (green), and Hoechst (blue). Images were taken at days 1 and 3 after bioprinting. Scale bar 1000 µm (trilaminar) and 200 µm (spheroids).

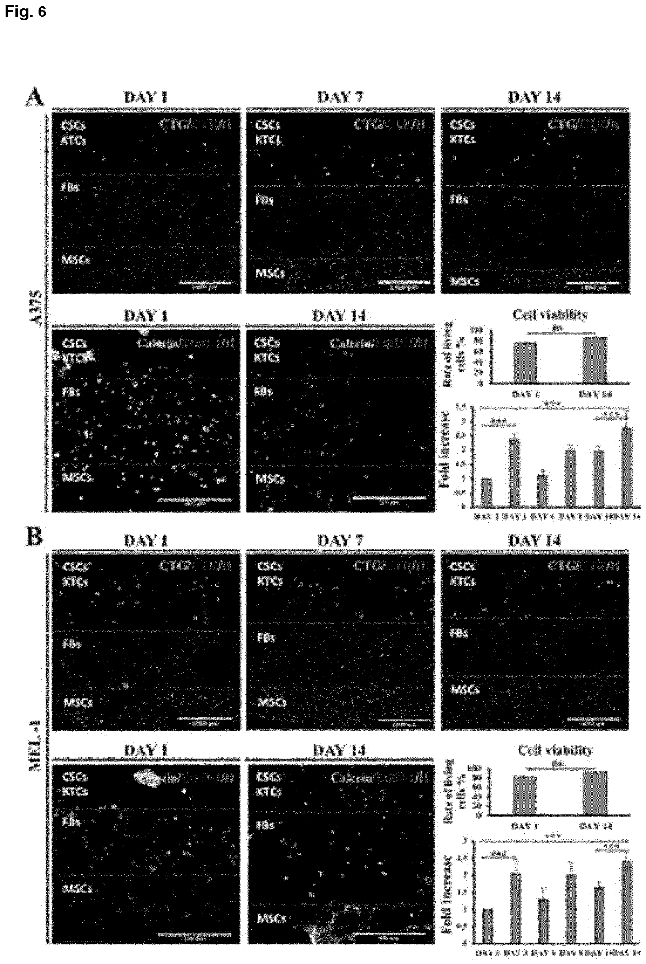

**Fig. 6****: Cellular structure, viability and proliferation analysis of the 3D bioprinted MM three-layered model including keratinocytes**. Confocal representative images obtained by confocal microscopy showing a cross-section of the 3D bioprinted A375 (A) and Mel-1 (B) MM models after 1, 7 and 14 days in culture. CSCs and MSCs were stained with CellTracker Green CMFDA (green) and KTCs and FBs were stained with CellTracker Red CMFDA (red) and Hoechst (blue). Below are shown the cross-section images at days 1 and 14 of the CSCs-KTCs-FBS-MSCs-laden hydrogels for (A) A375- and (B) Mel-1-based models. Scale bar: 500µm. Graphics on the top right show quantification of live/dead cells at days 1 and 14 with no significant difference on living cells fraction (n=4; p> 0,05). Graphics on the bottom right show fold change of proliferation of cells embedded in the trilaminar hydrogels at days 1, 3, 6, 8, 10 and 14 (n=4; p<0,005).

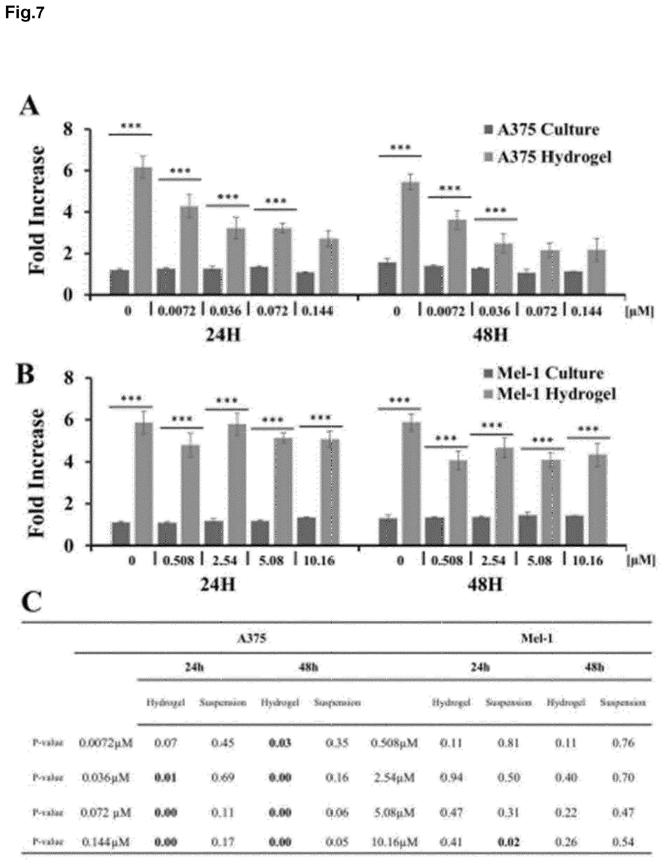

**Fig. 7****: Cytotoxic effect of vemurafenib in MM CSCs cultured as melanospheres in suspension and in the 3D bioprinted constructs**. Graphics show fold change of proliferation of A375 (A) and Mel-1 (B) CSCs cultured in suspension or embedded hydrogels after treatment with increasing doses of VMF for 24 and 48h (n=4; p<0,005). (C) Fold changes and P-values corresponding to graphics. P-Values in bold indicate statistical significance.

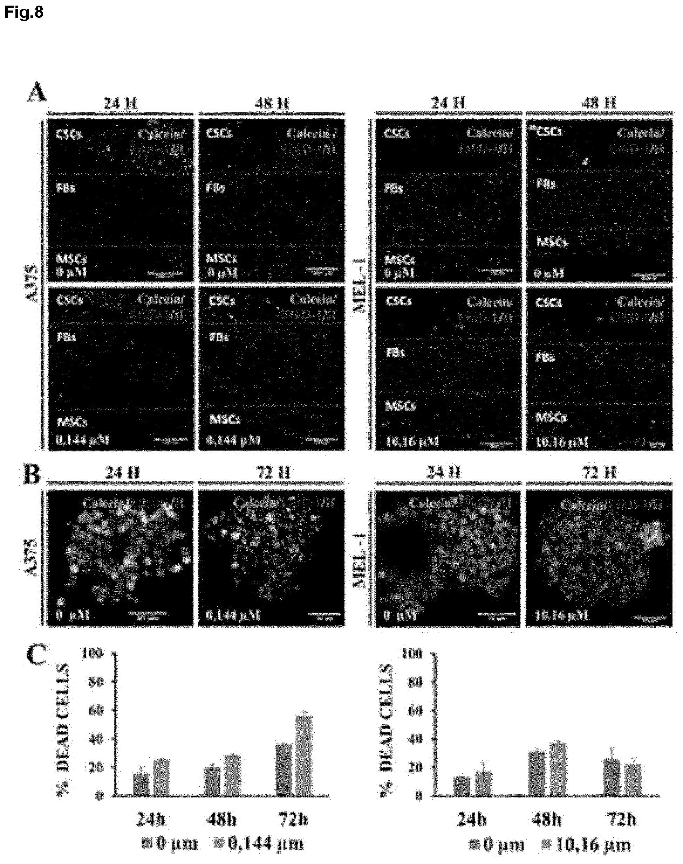

**Fig. 8****: Cytotoxic effect of vemurafenib in 3D bioprinted MM CSCs-based models.** (A) Confocal representative images showing a cross-section of the 3D bioprinted A375 and Mel-1 MM models after 24 and 48h in culture with different VMF doses: 0 µM, 0,144 µM (A375) and 0 µM, 10,16 µM (Mel-1). Scale bar: 1000µm. (B) Micrographs of the A375 or Mel-1 CSCs after 72h in culture with different VMF doses: 0µM, 0,144µM) (A375) and 0 µM, 10,16 µM (Mel-1). Scale bar: 500µm. (C) Graphics show quantification of live/dead CSCs percent at 24, 48 and 72h of both non-treated control and CSCs-based bioprinted hydrogels treated with high doses of VMF: 0,144µM (A375) and 10,16µM (Mel-1), respectively (n=4).

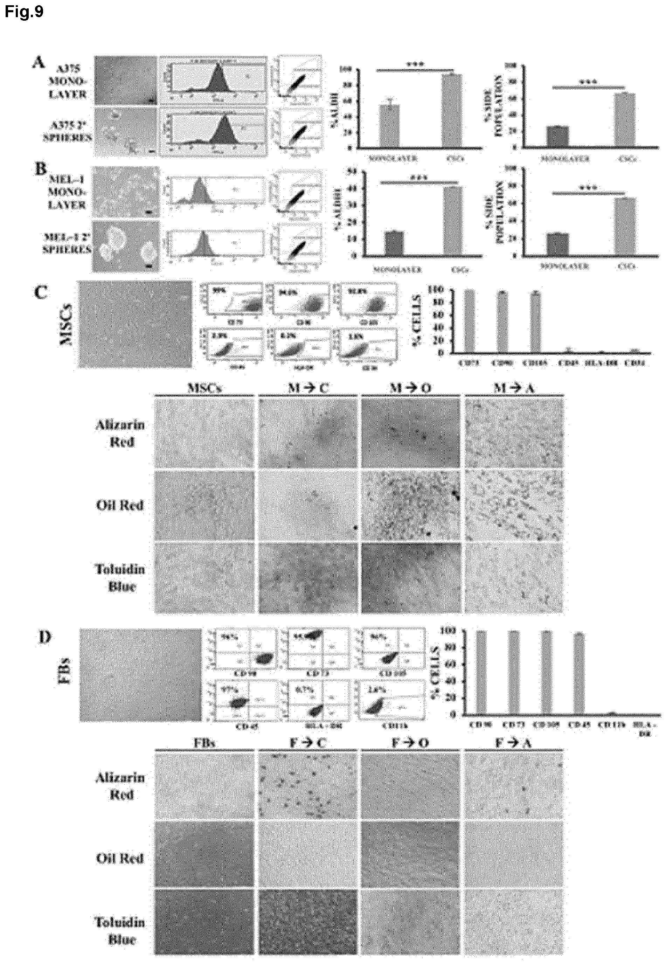

**Fig. 9****: Phenotypic and functional characterization of cell lines. (A-B)** Images of A375 (A) and Mel-1 (B) cells grown in monolayer and in suspension with serum-free spheres medium (10x). Scale bar: 50 µm. Percentage of ALDH1 activity and side population (SP) of cells grown in monolayer or in suspension with serum-free spheres medium (cytometry histograms besides). (C) Characterization of MSCs isolated from abdominal skin: MSCs show plastic adherence (Images obtained at 20x magnification). Cytometry histograms of MSCs characterization show positive expression of identifiable markers (CD105, CD73 and CD90) and negative expression for CD45, CD34 y HLA-II. (***p<0.005). (D) Characterization of FBs isolated from abdominal skin: FBs show plastic adherence (Images obtained at 20x magnification). Cytometry histograms of FBs characterization show positive expression of identifiable markers of FBs (CD105, CD73, CD45 and CD90) and negative expression for CD11b and HLA-II (***p<0.005). Also, it is shown the differentiation potential of MSCs (C) and FBs (D) towards adipogenic, osteogenic and chondrogenic lineage by induction with specific culture media, and subsequent staining with Oil Red O, Alizarin Red S and Toluidine Blue, respectively. First column pictures show negative controls, cells cultured in normal medium and histochemically stained. Original magnification: 20x

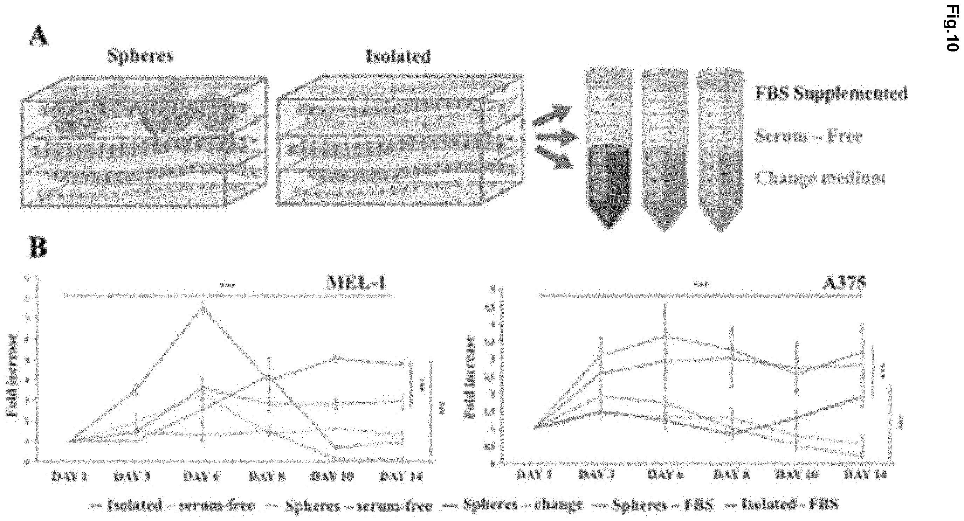

**Fig. 10****: Proliferation rates of CSCs with different culture conditions during 14 days.** (A) Scheme of the 5 culture conditions: i) CSCs spheres or CSCs individualized from secondary spheres bioprinted in the top layer, ii) living the other two cell-free hydrogel layers, iii) both cultured in serum-free sphere medium, iv) complete DMEM medium supplemented with FBS or v) with a change of medium at day 3. (B) Fold change of proliferation of Mel-1 and A375 CSCs embedded in 3D bioprinted hydrogels in spheres or individualized relative to the day 1, cultured with the different media conditions. Statistical significance is indicated as ***(p<0.005), **(p<0.01), *(p<0.05)

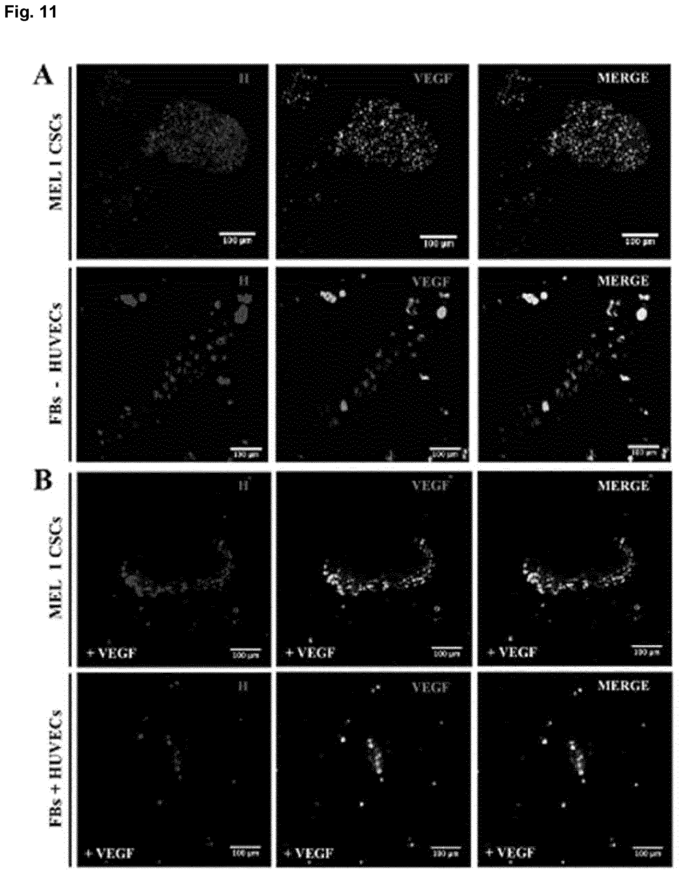

**Fig. 11****: Analysis of VEGF expression in the 3D bioprinted MM models by** (A) Confocal images of HUVEC-including trilaminar hydrogels, showing CSCs (top) and FBs mixed with HUVECs (bottom), both producing VEGF. (20x). (B) Confocal images of HUVEC-including trilaminar hydrogels treated with VEGF in the culture medium (250 ng/ml), showing CSCs (top) and FBs (bottom) expressing VEGF (20x). Scale bar: 100 µm.

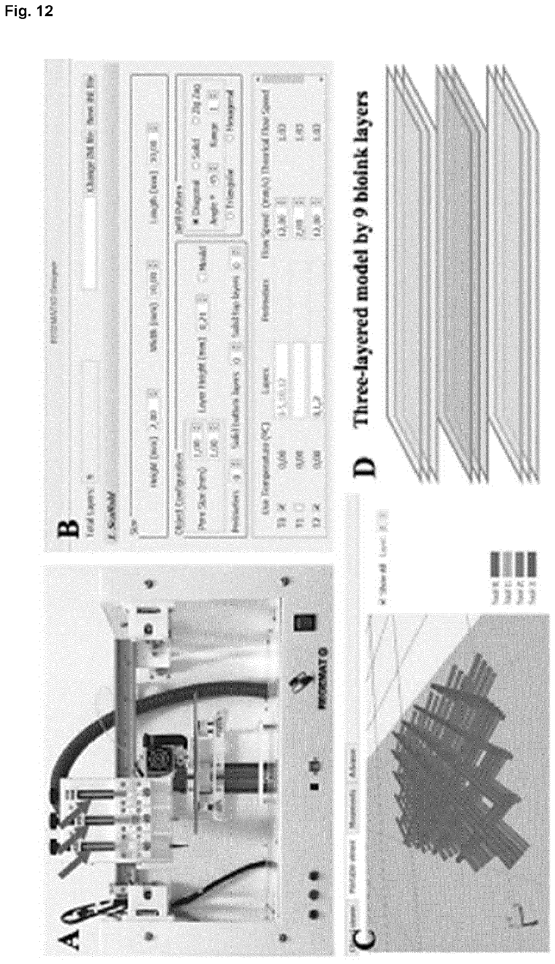

**Fig.12****: Parameters selected for the bioprinting procedure.** (A) Image of the REGEMAT 3D V1 bioprinter (red arrows indicate the thermal syringes). (B) Printing parameters chosen for the process included scaffold size (2 mm x 10 mm x 10 mm), pore size (0.6 mm), layer height (0.21 mm), diagonal pattern (45 °) and flow speed (12 mm/s). (C) Image of Regemat 3D's software showing the hydrogel design and its 9 layers disposed on a diagonal pattern. (D) Construct design of 9 layers composed of collagen and agarose and/or embedded cells.

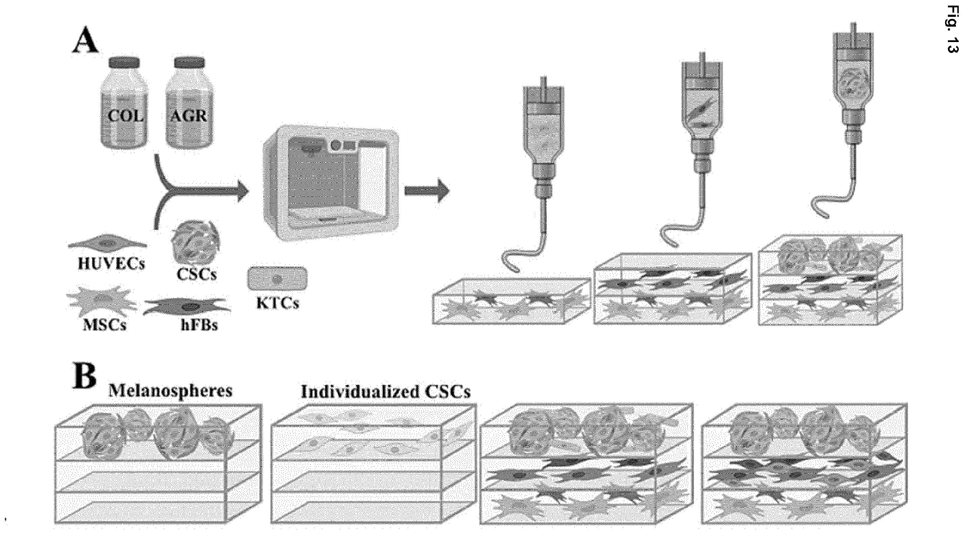

**Fig.13****: Schematic representation of the 3D bioprinted MM CSCs-based model.** (A) Bioprinting process scheme: Agarose-collagen type I bioink is mixed with the properly cell pellet and loaded into the thermal syringe. MSCs-laden hydrogel layer is printed first, then FBs and/or HUVECs-laden hydrogel layer, and finally the CSCs-laden and/or KTCs hydrogel layer. (B) Different constructs design, including: i) three-layered construct, with the individualized CSCs or conforming melanospheres CSCs-laden hydrogel in the top layer and cell-free hydrogel layers; ii) three-layered MM model including CSCs, KTCs, FBs and MSCs; iii) three-layered melanoma model including CSCs, HUVECs-FBs spheroids and MSCs.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus ten, preferably five, percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

### DESCRIPTION OF EMBODIMENTS

As explained in the examples, to closely mimic a MM environment, a trilaminar hydrogel was engineered, establishing the proper bioprinting protocol. First, MSCs (Mesenchymal stem cells) and FBs (Fibroblasts) were isolated from human skin tissue obtained during abdominoplasty surgery and correctly characterized (Fig. 9). Furthermore, CSCs from established A375 and primary patient-derived Mel-1 MM cell lines were obtained and characterized (Fig. 9). To obtain the 3D bioprinted MM models, a first layer of MSCs was printed followed by a layer of FBs, and a last one of Mel-1 or A375 MM CSCs; all these cells embedded in a mixture of 1.5% collagen type I and 1.5% agarose. Bioprinting process could affect the cell viability due to the high shear stress at the nozzle, so live/dead assay was performed at days 1 and 14 (Fig. 2). 24 hours after the bioprinting process cells displayed a high viability and homogenous distribution among the three layers with a remarkable staining in viable cells respect to death cells. Moreover, no significant differences on living cells percentages were found between day 1 and day 14.

Changes in mechanical properties of ECM are essential for tumor progression. Thus, we used rheological tests to measure the mechanical properties of the bioprinted agarose-collagen type I hydrogel construct over time in culture. The results can be found in the examples of the present specification and in the accompanying figures.

On the other hand, the vasculature is an essential element of the TME since it provides access to nutrients and oxygen and enables cell extravasation and metastasis. To study the induction of vasculature in the model, HUVECs (Human umbilical vein endothelial cells) and FBs co-cultured in spheroids were bioprinted on the dermal layer. Spheroids were generated using the hanging-drop technique, where the HUVECs form the nucleus of the spheroid, while the FBs are arranged in the second layer (Fig. 5A). HUVECs and CSCs were stained with CellTracker Red (CTR) dye and FBs and MSCs with CellTracker Green (CTG) dye, to easily trace the spatial disposition of the cells. In Figure 5B it can be seen that on the first day of culture the HUVECs-FBS spheres showed more compact, while on day 3 cells can be seen migrating from the spheroids, suggesting initial attempts of a vascularization process. Moreover, in our 3D bioprinted MM models it was not necessary the supplementation of VEGF when including HUVECs co-cultured with FBs in the dermal layer, as HUVECs, FBs and CSCs expressed VEGF in hydrogels cultured in VEGF-free medium, suggesting a cross-talk between cell types (Fig. 11).

In addition, the epidermal layer of the skin is basically composed of KTCs (keratinocytes), which are crucial for the functions of skin tissue. Therefore, in order to achieve a more biomimetic model, KTCs were included in the epidermal layer of the trilaminar hydrogel together with the MM CSCs (Fig. 6). The incorporation of this cell type did not modify the viability of our biofabricated MM constructs.

Furthermore, autologous models have shown high potential as a key step between research and clinical practice, as they can allow greater prediction of treatment success. Therefore, we tested the effect of VMF, a widely used drug for the treatment of MM patients, in the biofabricated A375 and Mel-1 CSCs-based models. This drug was chosen since both A375 and Mel-1 cell lines are BRAF mutated. Different doses of VMF were administered every 24 h on MM CSCs cultured in suspension and on CSCs embedded in the hydrogel, and cellular proliferation was tested after 24 and 48 h. Also, biofabricated trilaminar CSC-based models loaded with A375 or Mel-1 CSCs, FBs, and MSCs were treated in the same conditions, and cell viability was measured at 24, 48, and 72h with the Live/Dead kit (Fig. 7).

For the A375 CSCs-based model, the drug concentrations applied were 0µM, 0.0072µM, 0.036µM, 0.072µM and 0.144µM (Fig. 7A). In both non-treated and treated samples, CSCs proliferation significantly increased in hydrogel-embedded CSCs in comparison to melanospheres suspension cell culture at 24 and 48h (Fig. 7A; Table 1). For hydrogel-embedded CSCs the proliferation ratio decreased in a dose-dependent manner, whereas for CSCs cultured in suspension the lower dose (0.0072µM) inhibited the growth rate, and an increase of the dose did not improve the anti-tumor effect (Fig. 7C; Table 1). Furthermore, the viability assay demonstrated that the 3D bioprinted A375 CSCs-based model showed the antitumor effect of VMF on CSCs (Fig. 8). In the dermal and hypodermal layers, it was observed a strong viability marking (green), so FBs and MSCs viability was not affected by the drug treatment. In addition, a significant increase in the percentage of dead A375 CSCs was observed at 72h of treatment with 0.144µM VMF, with values up to approximately 50% with respect to the drug-free control (Fig. 8B and C).

On the other hand, for the Mel-1 CSCs-based model the concentrations of drug applied were 0µM, 0.508µM, 2.54µM, 5.08µM, and 10.16µM (Fig. 7B). Similarly, to the A375 CSCs-based model, a greater proliferation of CSCs in both non-treated and treated hydrogels was also observed in comparison to melanospheres cultured in suspension (Fig. 7B; Table 1). But contrarily to the A375 CSCs-based model, no significant differences were found between non-treated and treated samples either 24 or 48h (Fig. 7C; Table 1). Furthermore, there was no a significant decrease in the percentage of dead tumor cells at 24, 48, and 72h with respect to day 0 and the drug-free control biofabricated trilayered Mel-1 CSC-based model, and it remains approximately below 30-40% in all cases (Fig. 8C). These results indicate that VMF, even at high doses, did not compromise cell viability and proliferation for Mel-1 CSCs- based model.

Therefore, a first aspect of the invention is directed to a method, preferably *in vitro,* for generating a trilaminar 3D-bioprinted malignant skin model, preferably a trilaminar 3D-bioprinted Malignant melanoma skin model, comprising the steps of:
a) preparing a hydrogel solution (a pre-hydrogel solution) comprising agarose and collagen, preferably collagen type I, wherein the prehydrogel solution is maintained in a liquid state,
b) preparing a first, a second and a third bioink solutions by mixing a hydrogel solution according to step a) with:
   i. a cell population comprising mesenchymal stem cells, and optionally adipocytes, to provide the first bioink (to provide for the hypodermal layer),
   ii. a population comprising fibroblasts and, optionally, umbilical vein endothelial cells, endothelial cells and/or endothelial progenitor cells, to provide the second bioink (to provide for the dermal layer),
   iii. a population comprising one cell type of malignant melanoma and, optionally, keratinocytes, and optionally melanocytes, Langerhans' cells and/or Merkel's cells, to provide the third bioink (to provide for the epidermal layer), and
c) generating an artificial skin by 3D printing at least one layer of the first bioink, at least one layer of the second bioink on top of the last layer of the first bioink and at least one layer of the third bioink on top of the last layer of the second bioink layer,
wherein each of the bioink layers is jelled prior or simultaneously to 3D printing a further layer on top of the last printed layer, and wherein preferably each of the bioink solutions are preloaded into a thermal syringe for generating the artificial skin by 3D printing.

Optionally, the trilaminar 3D-bioprinted Malignant skin model could replace the malignant melanoma cells sited at the epidermal layer by other types of cancer stem cells from the skin such as squamous cell tumor cells or basal cell tumor cells.

It is noted that the above methodology for generating a trilaminar 3D-bioprinted malignant skin model, preferably a trilaminar 3D-bioprinted Malignant melanoma skin model, might also be alternatively formulated to comprise the following step:
a) generating an artificial skin by 3D printing at least one layer of a first bioink, at least one layer of a second bioink on top of the last layer of the first bioink and at least one layer of a third bioink on top of the last layer of the second bioink layer,

wherein each of the bioink layers is jelled prior or simultaneously to 3D printing a further layer on top of the last printed layer; and
wherein the first, second and third bioink solutions respectively comprise a i) hydrogel solution comprising agarose and collagen, preferably collagen type I, maintained in a liquid state, and mixed with ii):
   i. a cell population comprising mesenchymal stem cells, and optionally adipocytes, to provide the first bioink,
   ii. a population comprising fibroblasts and, optionally, umbilical vein endothelial cells, endothelial cells and/or endothelial progenitor cells, to provide the second bioink, and
   iii. a population comprising one cell type of malignant melanoma and, optionally, keratinocytes, and optionally Melanocytes, Langerhans' cells and/or Merkel's cells, to provide the third bioink, and
wherein preferably each of the bioink solutions are preloaded into a thermal syringe for generating the artificial skin by 3D printing.

The term "bioink" as used herein refers to a hydrogel with a mixture of cells with a hydrogel used for bioprinting. Therefore, a bioink composition may be applied for bioprinting and spraying. The term "3D bio-printing" used herein refers to the utilization of 3D printing like techniques to combine cells, growth factors, and biomaterials to fabricate a 3D object, part, tissue or structure that maximally imitate natural and biological characteristics. Preferably, 3D bio-printing utilizes the layer-by-layer method to deposit materials or bioinks to create structures that are later used in biotechnology, medicine and tissue engineering fields. The term "cell spraying" used herein refers to the technique of spraying cell on cell culture plates with a pump-action aerosol nozzle.

As is known to the expert in the art the composition of the bioink may contain several other components that find use in the change of the bioink characteristics which are required in the process of bioprinting or in the final product. Therefore, in a preferred embodiment of the first aspect of the invention, the trilaminar 3D-bioprinted malignant skin model may further comprise at least one of the components selected from a list consisting of alginate, gelatin from several origins (fish, bovine, porcine skin), fibrinogen from several origins (bovine, porcine, human plasma), intraarticular sodium hyaluronate, silk fibroin and sericine, xanthan, genipin, agar, chitosan, nanocellulose, proteoglicans (dermatan sulphate, hyaluronic acid, keratin,chondroitin sulphate, keratan sulphate), elastin, and other collagen types such as collagen II, III y IV, or their methacryloyl variants, or any combination thereof.

It is noted that the term "about", as used herein, refers to +/- 30%, preferably +/- 20%, preferably +/-15%, more preferably +/- 10%, of the indicated referred value.

In a preferred embodiment of the first aspect of the invention or of any of its preferred embodiments of the first aspect of the invention (including the above alternative aspect), each of the bioink layers has preferably a height of 0.15-0.4 mm; it is herein noted that the final trilaminar 3D-bioprinted malignant skin model, preferably a trilaminar 3D-bioprinted Malignant melanoma skin model, should preferably imitate the thickness or hight of the human skin of about 0.4 mm to about 1 cm, preferably about 0.4 mm to about 7 mm, more preferably 0.4 mm to about 4 mm.

In another preferred embodiment of the first aspect of the invention or of any of its preferred embodiments, the artificial skin by 3D printing comprises at least three layers of the first bioink, at least three layers of the second bioink on top of the last layer of the first bioink and at least three layers of the third bioink on top of the last layer of the second bioink layer.

In another preferred embodiment of the first aspect of the invention or of any of its preferred embodiments, each of the bioink solutions is preloaded into a thermal syringe prior to the 3D printing process, wherein said thermal syringe is preferably maintained at a temperature between 37 and 43°C, preferably between 38 and 42°C, more preferably about 40°C.

It is noted that the hydrogel solution, maintained in a liquid state, of the present invention can be easily prepared as indicated in the examples of the present invention by producing an agarose solution by dissolving agarose powder in a suitable medium such as a buffer such as PBS. The solution is then maintained at temperatures of about 4°C, and pre-heated at about 40°C before used, in order to keep it liquid for the printing process. On the other hand, collagen, preferably collagen type I, solution (for example 3.58 mg/mL rat tail collagen, Corning) can be maintained at about 4°C before used, and preferably neutralized with 1M NaCHO₃ just before printing. To prepare the bioink, volumes of agarose and collagen are mixed to obtain the desired concentrations of agarose and collagen, for example about 1.5% agarose and 1.5% collagen concentrations, at preferably a volumetric ratio of about 1:5 to about 1:1 (agarose:collagen), preferably a volumetric ratio of about 1:1 (agarose:collagen).

Therefore, in another preferred embodiment of the first aspect of the invention or of any of its preferred embodiments, the final concentration of agarose in the hydrogel solution of the invention is between 0.1% and 5% (w/v), preferably between 1% and 3% (w/v), preferably 1.5%, and the final concentration of collagen, preferably collagen type I preferably neutralized with NaCHO₃ or any alternative suitable base such as NaOH o KOH, is between 0.2% and 20% (w/v), preferably between 1% and 3% (w/v), preferably 1.5%. The terms "% by weight" or "% wt" or "%w/v" are considered equivalents and can be used interchangeably herein and refer to the weight percentage relative to the total weight or volume of the solution or dispersion, unless otherwise specified. It is further noted that the hydrogel of the present invention can optionally further comprise proteoglycans.

It is noted that the MSCs used in the first bioink, preferably a primary culture of human mesenchymal stem cells, can be isolated from bone marrow, placenta, umbilical cord blood, adipose tissue, adult muscle, corneal stroma, amniotic fluid, endometrium and dental tissues. In another preferred embodiment of the invention the mesenchymal stem cells are isolated from adipose tissue. Additionally, the first bioink may alternatively further comprise embryonic stem cells (ESCs), non-embryonic or adult stem cells, progenitor cells, and/or iPS cells.

The term "hydrogel" used herein refers to a 3D network or scaffold of mixture of materials, molecules, polymers or substances that are combined by chemical bonds, including covalent, ionic and supramolecular bonds, or by any combination thereof, to form water-swellable but water-insoluble structures with a solid, semi-solid or semi-liquid texture, which is used as tissue-engineering scaffold.

The term "prehydrogel" used herein refers to the mixture of materials, molecules, polymers or substances optionally combined with the cells that will form the hydrogel, before undergoing a phase change from liquid to gel.

The term "adipocytes" used herein refers to cells that make up adipose tissue. They are rounded cells, from 10 to 200 microns, with a lipid content that represents 95% of the cell mass and that forms the constitutive element of fatty tissue. Their fundamental characteristic is that they store a large amount of fats (triglycerides), which, in the case of adipocytes, white adipose tissue (the most abundant in the adult human body) are grouped together forming a large drop that occupies the majority of the cell.

The term "fibroblasts" used herein refers to is a resident cell type of the connective tissue. It synthesizes fibres and maintains the extracellular matrix of tissue in many animals. These cells provide a lattice-like structure (stroma) to many different tissues and play a crucial role in wound healing, being the most common cells of connective tissue. They are derived from primitive mesenchymal and pluripotent cells. Stromal cells that can potentially transform into fibroblasts, osteoblasts, adipocytes and muscle cells are identified in bone marrow cultures as adherent cells.

In another preferred embodiment of the first aspect of the invention or of any of its preferred embodiments, the population of mesenchymal stem cells are a primary culture of human mesenchymal stem cells, preferably a primary culture of human mesenchymal stem cells isolated from bone marrow, placenta, umbilical cord blood, adipose tissue, adult muscle, corneal stroma, amniotic fluid, endometrium and dental tissues. In another preferred embodiment of the invention the mesenchymal stem cells are isolated from adipose tissue.

In another preferred embodiment of the first aspect of the invention or of any of its preferred embodiments, the population of fibroblasts cells are a primary culture of human fibroblasts.

In another preferred embodiment of the first aspect of the invention or of any of its preferred embodiments, the population comprising one cell type of malignant melanoma comprises primary patient-derived cell line Mel-1 (BBSPA-Mel#1), A375 ATCC^{®} CRL-1619 cell line, or a mixture of both.

In another preferred embodiment of the first aspect of the invention or of any of its preferred embodiments, the optional population of umbilical vein endothelial cells of the second bioink are human umbilical vein endothelial ATCC^{®} CRL-1730TM.

In another preferred embodiment of the first aspect of the invention or of any of its preferred embodiments, the optional population of keratinocytes are human epidermal keratinocytes.

On the other hand, as a person skilled in the art knows, the trilaminar 3D-bioprinted malignant skin model of the invention can be comprised in a composition. So, in another aspect, the present invention relates to a composition, hereinafter "composition of the invention", comprising the trilaminar 3D-bioprinted malignant skin model of the invention as obtained or obtainable by the method of the first aspect of the invention or by any of its preferred embodiments.

A further aspect relates to the trilaminar 3D-bioprinted Malignant Model obtained or obtainable by the method of the first aspect of the invention or by any of its preferred embodiments.

Lyophilization of the trilaminar 3D-bioprinted Malignant Model obtained according to any of the above two aspects may be carried out or done in order to obtain a stable preparation of the same. The term "lyophilizing" or "lyophilization" as used herein refers to the rapid freezing and dehydration of the frozen product under high vacuum and is also commonly referred to as "freeze-drying".

The 3D-bioprinted Malignant Model obtained according to any of the above two aspects including its composition or lyophilized form finds several uses and applications. Accordingly, a further aspect of the present invention is the 3D-bioprinted Malignant Model of the invention for use in pharmacological development and preclinical screening of chemotherapeutic drugs, and basic study of the fundamental biology of cancer and the processes that mediate tumor proliferation, growth, metastasis and invasion.

The 3D-bioprinted Malignant Model or the composition of the invention can be used as in vitro disease model of skin pathologies, injuries, or tumor. In addition, the 3D-bioprinted Malignant Model of the present invention or the composition of the invention can be comprised in an implant to form a tissue scaffold that can be implanted into a study subject, such as mice, for use in pharmacological development and preclinical screening of chemotherapeutic drugs, and basic study of the fundamental biology of cancer and the processes that mediate tumor proliferation, growth, metastasis and invasion.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### EXAMPLE

### - Materials and Methods

### 4.1 Cell cultures

The human MM established cell line A375 (ATCC^{®} CRL-1619) and human umbilical vein endothelial cells (HUVEC) (ATCC^{®} CRL-1730TM) were obtained from American Type Culture Collection. The adult human epidermal keratinocytes (KTCs) were obtained from ScienCell (ScienCell; number 2110). The human melanoma primary patient-derived cell line Mel-1 comes from a malignant metastatic melanoma (stage M1a) skin biopsy and was provided by the Biobank of the Andalusian Public Health System (Spain). This cell line is hypotriploid (complex karyotype with multiple numerical and structural chromosome abnormalities), MelA-positive, p53-positive and S100-positive, and has high tumorigenic ability. The human mesenchymal stem cells (MSCs) and human fibroblasts (FBs) were isolated by an enzymatic digestion using collagenase I (Sigma Aldrich) from skin tissue during abdominoplasty surgery (ethics committee reference: 02/022010 Hospital Virgen de la Victoria, Málaga) after obtaining written informed consent. MSCs, FBs, Mel-1 and A375 cell lines were cultured with Dulbecco's Modified Eagle Medium (DMEM; Sigma Aldrich) supplemented with 10% Fetal Bovine Serum (FBS) (Gibco) and 1% penicillin/streptomycin (Sigma Aldrich) in culture flask under controlled conditions (37°C, 5% CO2). HUVECs were cultured with endothelial cell growth medium 2 (ECGM2; PromoCell) supplemented with Supplement Mix (PromoCell) in gelatin-coated culture flask. KTCs were cultured with keratinocyte medium (KM; ScienCell) supplemented with Keratinocyte Growth Supplement (ScienCell). At 80% of confluence, cells were subcultured using trypsin at 0.25% (Sigma).

### 4.2 Melanoma secondary spheres formation

A375 and Mel-1 cells were washed with PBS and 150.000 cell/well plate were resuspended in ultra-low adherence 6-well plates (Corning) with free-serum spheres culture medium composed by DMEM:F12 (Sigma Aldrich), 1% penicillin/streptomycin (Sigma), B27 (Gibco), 10 µg/mL Insulin-Transferrin-Selenium (ITS) (Gibco) 1 µg/mL Hydrocortisone, 4 ng/mL Heparin (Sigma Aldrich), 10 ng/mL Epidermal growth factor (EGF) (Sigma Aldrich), 10 ng/mL Fibroblast growth factor (FGF) (Sigma Aldrich), 10ng/mL Interleukin-6 (IL-6) (Miltenyi Biotec) and 10ng/mL Hepatocellular growth factor (HGF) (Miltenyi Biotec). To obtain the secondary spheres, cells from primary spheres were collected by centrifugation, and then dissociated with trypsin 0.25% (Sigma Aldrich). 150,000 single cells were plated and resuspended in serum-free spheres culture medium in ultra-low adherence 6-well plates (Corning).

### 4.3 Cell characterization

MSCs, FBs and CSCs were characterized by flow cytometry. MSCs and FBs were trypsinized, washed and resuspended in PBS with 1% bovine serum albumin (BSA; Sigma-Aldrich, and stained with fluorochrome-conjugated monoclonal antibodies for CD73, CD90, CD105, CD34, CD45 and HLA-DR (Miltenyi Biotec) according to manufacturer's instructions. Cells were analyzed by flow cytometry in a FACSCanto II cytometer (BD Biosciences). To characterize CSCs subpopulations enrichment, Hoechst efflux and ALDH1 activity were analyzed following the Side Population Protocol 53 and the ALDEFLUOR kit-manufacturing instructions (Stem Cell Technologies).

MSCs and FBs isolated from abdominal skin were cultured with specific inductive media. For adipogenic, osteogenic and chondrogenic differentiation, cells were cultured for two weeks in Adipogenic MSCs Differentiation Bullet Kit, Osteogenic MSCs Differentiation Bullet Kit (Lonza) and NH ChondroDiff Medium (Miltenyi Biotec), respectively. Differentiated cell cultures were stained with Oil Red O (Sigma) for adipogenic differentiation, Alizarin Red (Lonza) for osteogenic differentiation or Toluidine Blue (Sigma) for chondrogenic differentiation.

### 4.4 Cell-laden hydrogel fabrication

Agarose solution 3,3% (w/v) was prepared by dissolving Agarose UltraPureTM Low Melting Point (Thermo ScientificTM) powder in PBS. The solution was maintained at 4°C, and pre-heated at 40°C before used, in order to keep it liquid for the printing process. Collagen type I solution (3.58 mg/mL rat tail collagen, Corning) was maintained at 4°C before used, and neutralized with 1M NaCHO₃ just before printing. To prepare the bioink, properly volumes of agarose and collagen were mixed to obtain 1.5% agarose and 1.5% collagen concentrations at a volumetric ratio 1:1.

### 4.4.1 CSCs- based bioink for proliferation assay

Secondary spheres were collected on PBS and centrifuged. Isolated CSCs were obtained by harvesting spheres with trypsin-EDTA (0.25 %, Sigma Aldrich) in order to dissociate spheres into single cells. Each pellet was carefully mixed with collagen type I and agarose homogenously. The final cell-laden bioink was then loaded into a sterile 3 mL thermal syringe, previously heated at 37 °C to avoid a thermal shock.

### 4.4.2 Cell-laden bioinks for MM models

MSCs, FBs and KTCs in culture flasks were harvested with trypsin (0.25%, Sigma Aldrich), and centrifuged. CSCs spheroids were also collected and centrifuged. For structure analysis CSCs, FBs, KTCs and MSCs were stained with the fluorescent dye CellTrackerTM Green CMFDA (Invitrogen Inc., Carslbad, CA, USA) or CellTrackerTM Red CMFDA (Invitrogen 504 Inc., Carslbad, CA, USA), following manufacturer's instructions before the bioprinting process. After centrifugation, cell pellets were loaded into the syringe as described in section 4.4.1. Cells concentration for all the models were: CSCs spheres (cell density of 3x106 cells/mL), KTCs (cell density of 3x106 cells/mL), FBs (cell density of 1,5x106 cells/mL), HUVECs (cell density of 1,5x106 cells/mL) and MSCs (cell density of 1,5x106 cells/mL) placed in different contiguous layers (from top to bottom) simulating the three-layered MM skin structure, so loaded in their correspondents' syringes.

### 4.4.3 Cell-laden bioink for vascularizated MM models

Isolated HUVECs and FBs or HUVECS-FBs spheroids were printed in the middle layer. HUVECs-FBs spheroids were performed following the hanging-drop method, and cells were previously stained as described in section 4.4.2. Briefly, HUVECs were seeded at concentration of 500 cells/drop in ECGM2 with 2,4 mg/ml methyl cellulose (Sigma Aldrich). After 24h FBs were seeded at concentration of 500 cells/drop over the HUVECs spheres. After 24h, HUVECs-FBs spheroids were collected and centrifuged and, then, cell pellets were loaded into the syringe as described in section 4.4.1.

### 4.4 Bioprinting procedure

Cell laden hydrogels were bioprinted by an extrusion-based REGEMAT 3D V1 bioprinter (REGEMAT 3D, Granada, Spain). Bioprinting parameters were programmed employing the Regemat 3D's software: flow speed 12 mm/s, pore size 0.6 mm2, 9 layers (height 0.21 mm), infill pattern diagonal (45°), width 10mm, length 10mm, syringe temperature 40°C (Fig. 12). For extrusion, a plastic needle (TT22-DHUV-1000) was used.

Different models were designed and bioprinted in this study, as shown in figure 13: i) CSCs-loaded hydrogel both as melanospheres or individualized; ii) trilaminar hydrogels including CSCs, FBs, MSCs and KTCs, iii) Vascularized hydrogels loading HUVECs-FBs spheroids on the dermal layer. Once prepared the different bioinks and loaded into the thermal syringes, they were placed into the bioprinter and the process was initiated. The printed constructs quickly gelled after printing and were placed into ultra-low adherence 12-well plates (Corning) and maintained for 14 days under cell culture conditions at 37°C in a 5% CO2 atmosphere. 1 mL of corresponding fresh medium was added and replaced every 3 days.

### 4.5 Hydrogel structure analysis

In order to assess the maintenance of the three-layer structure, CSCs, FBs, KTCs and MSCs were stained before printing as previously described in section 4.4.2. Images were obtained by confocal microscopy (Nikon Eclipse Ti-E A1, USA) the days 1, 7 and 14 after bioprinting.

### 4.6 Proliferation assays

Cell proliferation was measured using Alamarblue^{®} assay (Bio-Rad Laboratories, Inc., manufactured by Trek Diagnostic Systems. U.S.) on days 1, 3, 6, 8, 10 and 14 after bioprinting. 3D bioprinted hydrogels were incubated with 10µl/100µL of Alamarblue^{®} solution for 3 h at 37°C. At this time, fluorescence intensity was measured at an excitation wave length of 530 nm and emission of 590 nm (Microplate Reader MB-580/530, Heales).

### 4.7 Cell viability assays

Cell-laden 3D bioprinted hydrogels were stained with the fluorescent Live/Dead Kit (ThermoFisherTM,) following the manufactures indications. Images were taken by confocal microscopy (Nikon Eclipse Ti-E A1, USA) and analyzed using Image J Software. Live/dead cells were counted using Image J Software in 5 random sights of magnifying at 10x for 4 replicates of each sample.

### 4.8 ESEM images

Cell-laden and cell-free hydrogels were fixed with glutaraldehyde 2.5% (Merck) during 1h at 4°C and washed and maintained with Sodium Cacodylate Buffer 0.1M (EMS Electron Microscopy Science). For critical point the samples were then maintained with Osmium tetroxide 1% RT during 1h and dehydrated in a series of ethanol solutions (50%, 70%, 90%, 100%, 100%, 100%), by soaking the samples in each solution for 15 min. Subsequently, samples were critical point dried (Anderson, 1951) in a desiccator (Leica EMCPD300), and covered by evaporating them in a carbon evaporator (Emitech K975X). Hydrogels were imaged with the Environmental Scanning Electron Microscope (QuemScan650F).

### 4.9 Rheology

The rheological tests were carried out in a torsional rheometer MCR302 (Anton Paar, Austria) at 25 °C. The hydrogels were measured using plate-plate geometry with 20 mm diameter and 5 mm gap. Each sample was placed on top of the lower base of the rheometer, the rheometer head was displaced downwards at a constant velocity of 10 µm/s. Once the head registers a normal force larger than 0.5 N, the rheometer head stops and the normal force is kept constant at 0.5 N for 30 more seconds. Finally, the sample is subjected to an oscillatory shear of 0.1% strain amplitude and an excitation frequency of 1 Hz at a constant normal force of 0.5 N to quantify the viscoelastic properties.

### 4.10 Vasculature assays

For VEGF-effect evaluation, tri-laminar 3D bioprinted MM hydrogels containing isolated HUVECs and FBs were kept in medium supplemented with VEGF (250 ng/mL) or without VEGF for two weeks. Melanoma models were fixed in 4% paraformaldehyde at room temperature for 1 hour, preserved in 30 % sucrose at 4 °C overnight, and soaked in OCT. The OCT blocks were cut into 8 µm sections and analyzed immunohistochemically. Samples were permeabilized with 0.1 % Triton X-100 (Sigma) for 15 min and blocked with 3 % BSA for 1 h at room temperature, and then incubated with anti-VEGF (Santa Cruz Biotechnology, sc-152) antibody (1:100) overnight at 4° C. Next day, samples were washed thrice with PBS, incubated with anti-VEGF secondary antibody (Alexa-Thermofisher) (1:500) for 1 hour at room temperature, washed with PBS and stained with Hoechst 33342 (Sigma Aldrich). Finally, samples were mounted in Aqueous Mounting Medium (Santa Cruz Biotechnology). Images were taken by confocal microscopy (Nikon Eclipse Ti-E A1, USA) and analyzed 583 using Image J Software.

### 4.11 Effect of vemurafenib in the tumor model

The drug VMF was tested on CSCs in culture and embedded in the hydrogels for the two tumor cell lines, A375 and Mel-1, following the protocol described in section 4.5. It was also tested in the 3D biofabricated trilaminar models loaded with CSCs, FBs and MSCs, and a cell viability analysis was performed following the protocol described in section 4.7. The doses administered for the A375 cell line were 0 µM, 0.0072 µM, 0.036, 0.072 µM and 0.144µM. The doses administered for the Mel-1 cell line were 0 µM, 0.508 µM, 2.54 µM, 5.08 µM, and 10.16µM. The drug was refreshed every 24h.

### 4.12 Statistical analysis

Results presented in this work are represented as mean ± SEM from at least three replicas. ANOVA test was used to determine data normality. Two-tailed Student's t-test was used to determine test significance between different hydrogels or conditions. Results were considered statistically significantly different at *p < 0.05, **p < 0.01 and ***p < 0.005.

### - Results

### 2.1 Cell viability and characterization of trilaminar MM models

To closely mimic a MM environment a trilaminar hydrogel was engineered, establishing the proper bioprinting protocol. First, MSCs and FBs were isolated from human skin tissue obtained during abdominoplasty surgery and correctly characterized (Fig. 9). Furthermore, CSCs from established A375 and primary patient-derived Mel-1 MM cell lines were obtained and characterized (Fig. 9), and the optimal culture condition of these CSCs within the hydrogel was defined (Fig. 10).

To obtain the 3D bioprinted MM models, a first layer of MSCs was printed followed by a layer of FBs, and a last one of Mel-1 or A375 MM CSCs; all these cells embedded in a mixture of 1.5% collagen type I and 1.5% agarose. To test the degradation, images of the bioprinted tumor were taken at days 1 and 14 showing that hydrogels maintained a length side of 1.2 cm, showing no modifications in the shape during two weeks (Fig. 1A). In order to follow the maintenance of the layered structure, CSCs and MSCs were pre-stained with CellTracker Green CMFDA (CTG) dye to visualize epidermal and hypodermal layers, and FBs with CellTracker Red CMFDA (CTR) to stain the dermal layer. Confocal micrographs showed that the 3D bioprinted constructs were able to maintain their trilaminar structure over time (Fig. 1B), evidencing the correct consistence and desirable properties of the agarose- and collagen type I-based bioink.

Bioprinting process could affect to cell viability due to the high shear stress at the nozzle, so live/dead assay was performed at days 1 and 14 (Fig. 2). 24 hours after the bioprinting process cells displayed a high viability and homogenous distribution among the three layers with a remarkable staining in viable cells (green) respect to death cells (red). Moreover, no significant differences on living cells percentages were found between day 1 and day 14. To corroborate these results, proliferation of trilaminar hydrogels was measured with Alamarblue^{®} until day 14 (Fig. 2). In both tumor models, statistical analysis showed a significant increment (***p< 0.005) on cell proliferation rate from day 1 forward. Mel-1 CSCs laden bioprinted hydrogels maintained a constant proliferation rate from day 3 to day 14 (Fig. 2A), while A375 CSCs laden bioprinted hydrogels showed a significant increase in proliferation rate at day 14 compared to day 10 (***p< 0.005) (Fig. 2B).

### 2.2 Ultrastructural hydrogel characterization

In order to characterize the microstructure of the bioprinted hydrogels, an environmental scanning electron microscopy (ESEM) was performed after 14 days in culture. Although an irregular fibrillar pattern was observed on the surface (Fig. 3A1), after cutting transversely the hydrogel, a dense network of fibrils was discerned with porous of irregular shape ranging between 2-3 µm in diameter and fibers with a thickness of 70-140 nm (Fig. 3A2-3). Cell analysis showed that MM CSCs were forming spheres on the top hydrogel layer (epidermis) and MSCs and FBs were found on the middle and bottom layers (dermis and hypodermis, respectively) (Fig. 3B). Also, signs of intercellular communication were observed as showed extracellular vesicles (EVs) secretion (Fig. 3C), ranging from about 80 nm to vesicles of about 500 nm (Fig. 3C1, red arrows), sizes that corresponds with exosomes and microvesicles, which were found dispersed by the hydrogel (Fig. 3C2, red arrows). Furthermore, the model displayed high cell metabolic activity as suggest cell division (Fig. 4C2, white arrow), the production of a dense material that covered the fiber networks, indicating ECM secretion, and that the remodeling of their own extracellular microenvironments (Fig. 3C1, white arrow, and Fig.3C3).

### 2.3 Rheological properties of melanoma 3D model

Changes in mechanical properties of ECM are essential for tumor progression. Thus, we used rheological tests to measure the mechanical properties of the bioprinted agarose-collagen type I hydrogel construct over time in culture. Figure 4A illustrates the viscoelastic moduli of hydrogels containing cell-free layers, only one cell-laden layer: MSCs-layer, FBs-layer, CSCs spheres-layer (composed of Mel-1 or A375 cells); or containing the three cell-laden layers together, called trilaminar (MSCs, FBs, and CSCs spheres-layers). Independent on cell composition, the storage modulus of agarose-collagen type I hydrogel remained always larger than the loss modulus, evidencing the existence of a gel-like structure. Initially, the range was between 2666,772 and 5335,611 Pa for G' and 148 and 245 Pa for G", without significant differences among cell-type used and with cell-free hydrogels. All these values were comparable to those of healthy human skin tissue used as control (2945,767 Pa and 632 Pa for G' and G", respectively). At day 10, it was observed a significant decrease in viscoelastic moduli of CSCs-embedded hydrogels while in trilaminar and hydrogels embedding non-cancerous cell-layers this phenomenon was notable later (at day 20). The viscoelastic moduli from hydrogels containing only CSCs spheres-layer were recovered again by day 20, raising G' values of 3359,583 and 2666,111 Pa, for Mel-1 and A375 sphere, respectively. In Figure 4B, it is shown the compression moduli of the hydrogels with different cell composition. As it is observed, the incorporation of cells did not increase the Young moduli in the hydrogels, except for trilaminar. Similar to viscoelastic moduli, the values of hydrogels with different cell composition decreased significantly after culture period, being clearly faster in those containing CSCs spheres-layer. Measurement from trilaminar and hydrogels containing CSCs spheres-layer revealed significant differences in stiffness by day 10, exhibiting values of 5338,25 and 9089,16 Pa, respectively. These compression values were increased from day 10 to day 20, in the case of hydrogels containing only CSCs spheres-layer, although differences were not significant.

### 2.4 Induction of vascularization in the 3D bioprinted MM models.

The vasculature is an essential element of the TME since it provides access to nutrients and oxygen, and enables cell extravasation and metastasis. To study the induction of vasculature in the model, HUVECs and FBs co-cultured in spheroids were bioprinted on the dermal layer, as it has been shown that 3D structures improve the development of vascularization in in vitro models, and long-term stability of vasculature structures can be enhanced by including FBs. Spheroids were generated using the hanging-drop technique, where the HUVECs form the nucleus of the spheroid, while the FBs are arranged in the second layer (Fig. 5A). HUVECs and CSCs were stained with CellTracker Red (CTR) dye and FBs and MSCs with CellTracker Green (CTG) dye, to easily trace the spatial disposition of the cells. In Figure 5B it can be seen that on the first day of culture the HUVECs-FBS spheres showed more compact, while on day 3 cells can be seen migrating from the spheroids, suggesting initial attempts of a vascularization process.

Moreover, in our 3D bioprinted MM models it was not necessary the supplementation of VEGF when including HUVECs co-cultured with FBs in the dermal layer, as HUVECs, FBs and CSCs expressed VEGF in hydrogels cultured in VEGF-free medium, suggesting a cross-talk between that cell types (Fig. 11).

### 2.5 Incorporation of keratinocytes in the epidermal layer

The epidermal layer of the skin is basically composed of KTCs, which are crucial for the functions of skin tissue. Therefore, in order to achieve a more biomimetic model, KTCs were included in the epidermal layer together with the MM CSCs (Fig. 6). The incorporation of this cell type did not modify the viability of our biofabricated MM constructs. As can be seen in Figure 6 the trilaminar models are stable over 14 days and cell viability did not decrease for the three layers (p>0,05). Moreover, a significant increase of cell proliferation was observed over two weeks (p<0,005). These results indicate that KTCs were not affected in their viability and proliferation when they are encapsulated within the 3D bioprinted MM hydrogels.

### 2.6 Antitumor drug response of melanoma 3D model

Autologous models have shown high potential as a key step between research and clinical practice, as they can allow greater prediction of treatment success. Therefore, we tested the effect of VMF, a widely used drug for the treatment of MM patients, in the biofabricated A375 and Mel-1 CSCs-based models. This drug was chosen since both A375 and Mel-1 cell lines are BRAF mutated. Different doses of VMF were administered every 24 h on MM CSCs cultured in suspension and on CSCs embedded in the hydrogel, and cellular proliferation was tested after 24 and 48 h. Also, biofabricated trilaminar CSC-based models loaded with A375 or Mel-1 CSCs, FBs, and MSCs were treated in the same conditions, and cell viability was measured at 24, 48, and 72h with the Live/Dead kit (Fig. 7).

For the A375 CSCs-based model, the drug concentrations applied were 0µM, 0.0072µM, 0.036µM, 0.072µM and 0.144µM (Fig. 7A). In both non-treated and treated samples, CSCs proliferation significantly increased in hydrogel-embedded CSCs in comparison to melanospheres suspension cell culture at 24 and 48h (Fig. 7A; Table 1). For hydrogel-embedded CSCs the proliferation ratio decreased in a dose-dependent manner, whereas for CSCs cultured in suspension the lower dose (0.0072µM) inhibited the growth rate, and an increase of the dose did not improve the anti-tumor (Fig. 7C;
Table 1).

**Table 1:**

| | **A375** | | | | | | **Mel-1** | | | |
|---|---|---|---|---|---|---|---|---|---|---|
| | | **24h** | | **48h** | | | **24h** | | **48h** | |
| | Hydrogel Suspension Hydrogel Suspension | | | | | | Hydrogel Suspension Hydrogel Suspension | | | |
| **Fold** | 0µM | 6.15 | 1.2 | 5.45 | 1.57 | 0µM | 5.86 | 1.10 | 5.88 | 1.31 |
| **P-value** | | **0.00** | **0.01** | **0.00** | **0.02** | | **0.00** | 0.18 | **0.01** | 0.12 |
| **Fold** | 0.0072µM | 4.26 | 1.25 | 3.62 | 1.39 | 0.508µM | 4.81 | 1.08 | 4.7 | 1.35 |
| **P-value** | | **0.00** | **0.00** | **0.00** | **0.00** | | **0.00** | 0.11 | **0.00** | **0.00** |
| **Fold** | 0.036µM | 3.22 | 1.23 | 2.49 | 1.27 | 2.54µM | 5.78 | 1.17 | 4.67 | 1.39 |
| **P-value** | | **0.01** | 0.07 | **0.03** | **0.00** | | **0.00** | 0.06 | **0.00** | **0.00** |
| **Fold** | 0.072 µM | 3.22 | 1.37 | 2.16 | 0.92 | 5.08µM | 5.14 | 1.18 | 4.09 | 1.44 |
| ***P*-value** | | **0.00** | **0.00** | **0.03** | 0.65 | | **0.00** | **0.00** | 0.00 | 0.00 |
| **Fold** | 0.144µM | 2.7 | 1.07 | 2.17 | 1.16 | 10.16µM | 5.08 | 1.34 | 4.34 | 1.42 |
| **P-value** | | **0.01** | **0.03** | 0.10 | **0.00** | | **0.00** | **0.00** | **0.00** | **0.01** |

| | | | | | | | | | | |
|---|---|---|---|---|---|---|---|---|---|---|
| *P*-Values in bold indicate statistical significance | | | | | | | | | | |

Statistical values of CSCs proliferation rate respect to day 0.

Furthermore, the viability assay demonstrated that the 3D bioprinted A375 CSCs-based model showed the antitumor effect of VMF on CSCs (Fig. 8). In the dermal and hypodermal layers it was observed a strong viability marking (green), so FBs and MSCs viability was not affected by the drug treatment (Fig. 8A). In addition, a significant increase in the percentage of dead A375 CSCs was observed at 72h of treatment with 0.144µM VMF, with values up to approximately 50% with respect to the drug-free control (Fig. 8B and C).

On the other hand, for the Mel-1 CSCs-based model the concentrations of drug applied were 0µM, 0.508µM, 2.54µM, 5.08µM, and 10.16µM (Fig. 7B). Similarly to the A375 CSCs-based model, a greater proliferation of CSCs in both non-treated and treated hydrogels was also observed in comparison to melanospheres cultured in suspension (Fig. 7B; Table 1). But contrarily to the A375 CSCs-based model, no significant differences were found between non-treated and treated samples either 24 or 48h (Fig. 7C; Table 1). Furthermore, there was no a significant decrease in the percentage of dead tumor cells at 24, 48, and 72h with respect to day 0 and the drug-free control biofabricated trilayered Mel-1 CSC-based model, and it remains approximately below 30-40% in all cases (Fig. 8C). These results indicate that VMF, even at high doses, did not compromise cell viability and proliferation for Mel-1 CSCs- based model.

## Claims

1. An *in vitro* method for generating a trilaminar 3D-bioprinted Malignant melanoma skin model, comprising the steps of:
a) preparing a hydrogel solution comprising agarose and collagen, preferably collagen type I, wherein the hydrogel solution is maintained in a liquid state,
b) preparing a first, a second and a third bioink solutions by mixing a hydrogel solution according to step a) with:
i. a cell population comprising mesenchymal stem cells, and optionally Adipocytes, to provide the first bioink,
ii. a population comprising fibroblasts and, optionally, umbilical vein endothelial cells, endothelial cells and/or endothelial progenitor cells, to provide the second bioink,
iii. a population comprising one cell type of malignant melanoma and, optionally, keratinocytes, and optionally Melanocytes, Langerhans' cells and/or Merkel's cells, to provide the third bioink, and
c) generating an artificial skin by 3D printing at least one layer of the first bioink, at least one layer of the second bioink on top of the last layer of the first bioink and at least one layer of the third bioink on top of the last layer of the second bioink layer,
wherein each of the bioink layers is jelled prior or simultaneously to 3D printing a further layer on top of the last printed layer.

2. The method according to claim 1, wherein each of the bioink layers has preferably a height of 0.15-0.25 mm, and wherein each of the bioink solutions is preloaded into a thermal syringe prior to the 3D printing process, wherein said thermal syringe is preferably maintained at a temperature between 37 and 43°C, preferably between 38 and 42°C, more preferably about 40°C.

3. The method according to any of claims 1 or 2, wherein the final concentration of agarose in the hydrogel solution of the invention is between 1 and 3% (w/v), preferably 1.5%, and the final concentration of collagen, preferably collagen type I neutralized with NaCHO₃ or any alternative suitable base such as NaOH o KOH, is between 1 and 3% (w/v), preferably 1.5%.

4. The method according to any of claims 1 to 3, wherein the collagen of step a) is type I collagen neutralized with with NaCHO₃ or any alternative suitable base such as NaOH o KOH.

5. The method according to any of claims 1 to 4, wherein the population of mesenchymal stem cells are a primary culture of human mesenchymal stem cells.

6. The method according to any of claims 1 to 5, wherein the population of fibroblasts cells are a primary culture of human fibroblasts.

7. The method according to any of claims 1 to 6, wherein the population of at least one cell type of malignant melanoma comprises primary patient-derived cell lines.

8. The method according to any of claims 1 to 7, wherein the population of umbilical vein endothelial cells are present and are preferably human umbilical vein endothelial ATCC^{®} CRL-1730TM.

9. The method according to any of claims 1 to 8, wherein the population of keratinocytes are present and are preferably human epidermal keratinocytes.

10. A trilaminar 3D-bioprinted Malignant Model obtained or obtainable from the method of claims 1 to 9.

11. In vitro use of the trilaminar 3D-bioprinted Malignant Model obtained or obtainable from the method of claims 1 to 9 in pharmacological development and preclinical screening of chemotherapeutic drugs, and basic study of the fundamental biology of cancer and the processes that mediate tumor proliferation, growth, metastasis and invasion.
